# EUROPEAN PATENT APPLICATION

(11) **EP 1 923 007 A1**
(43) Date of publication of application: **21.05.2008**
(21) Application number: 06124263.2
(22) Date of filing: 16.11.2006
(51) Int. Cl.: A61B 17/16, A61B 17/17, A61B 17/02

(54) **Instrument**

(71) Applicant: Zimmer GmbH, 8404 Winterthur (CH)
(72) Inventor: Filippi, Michael, 8200, Schaffhausen (CH); Cicerchia, Guiseppe, 8406, Winterthur (CH); Heller, Mathias, 8352, Räterschen (CH)

(57) **Abstract**

The disclosure relates to an instrument comprising a tool unit (22) including a tool (9), and a sleeve unit (21) including a sleeve member (2) having a passage, said passage defining an instrument axis (16), wherein the tool unit (22) is suited for being slidably received within the passage of the sleeve member (2) and for positioning the tool (9) laterally offset with respect to the instrument axis (16), wherein the tool unit (22) has a working side and a manipulation side, the working side being adapted to pass through the passage of the sleeve member (2), and wherein the tool unit (22) is suited for arranging the tool (9) within the passage of the sleeve member (2) in at least two different rotational positions with respect to the instrument axis (16).

## Description

The present disclosure relates to an instrument, in particular for the reaming of two adjacent bodies, for example vertebral bodies.

Two adjacent vertebral bodies of a spine segment are by nature separated from one another by an intervertebral disc. Dysfunction of an intervertebral disc may cause back pain. Known surgical treatment of back pain encompasses techniques including vertebral disc replacement by an artificial vertebral disc and fusion of the vertebral bodies by a fusion cage packed with autogenous bone graft. After intervertebral disc excision, the two adjacent vertebral bodies are reamed to provide endplates to allow insertion of the artificial vertebral disc or the fusion cage.

Here, an instrument of the initially named kind is now proposed that, in addition to a plurality of other advantageous properties, is compact, easy to handle and requires only a small operating space. More specifically, it should, for example, be made possible to prepare endplates for implantation of an implant replacing the intervertebral disc, for example, an artificial vertebral disc or a fusion cage, without having to move the instrument or a part thereof in order to conduct the endplate preparation. In accordance with a further aspect, an instrument should be set forth which allows an anterior approach to the spine. In accordance with a further aspect, a method for the working of at least one bone, in particular for the reaming of two adjacent vertebral bodies, should be set forth.

In addition to other advantageous properties, the features described in the claims can also satisfy these demands.

The instrument as set forth comprises a tool unit including a tool, and a sleeve unit including a sleeve member having a passage, which passage defines an instrument axis. The tool unit is suited for being slidably received within the passage of the sleeve member and for positioning the tool laterally offset, in particular eccentrically, with respect to the instrument axis. The tool unit has a working side and a manipulation side, the working side being adapted to pass through the passage of the sleeve member. In addition, the tool unit is suited for arranging the tool within the passage of the sleeve member in at least two different rotational positions with respect to the instrument axis. This makes it possible to provide tooling action offset to the instrument axis. In particular, this makes it possible to prepare endplates for implantation of an implant replacing an intervertebral disc without having to move the sleeve member, which is positioned between two vertebral bodies in order to conduct the endplate preparation.

It should be noted that the use of the instrument is not limited to the reaming of two adjacent vertebral bodies but directed to the working of at least one body, e.g., bone, in general.

In one embodiment, the tool unit has a tooling direction in which tooling is effected, which tooling direction is inclined with respect to the instrument axis by not more than 15°, in particular not more than 10°. In particular, the tooling direction is inclined with respect to the instrument axis by not more than 5°, in particular not more than 2°. In particular, the tooling direction is essentially parallel to the instrument axis.

In a further embodiment, the tool unit comprises a guide member, said guide member having a guide member axis, wherein the guide member is suited for being slidably received within the passage of the sleeve member and for positioning the tool laterally offset with respect to the guide member axis. In particular, the guide member is interposed between the tool and the sleeve member, and the tool and the sleeve member are interconnected by the guide member, respectively.

In a further embodiment, the tool comprises a tool axle, which is suited for being received within the guide member laterally offset with respect to the guide member axis (23). In particular, the tool axle is rotatably and/or slidably received within the guide member. The tool axle can have a circular cross-section or any other, e.g., square or rectangular, non-circular cross-section.

In a further embodiment, the tool unit has a tooling direction in which tooling is effected, wherein the tool axle comprises a longitudinal axis, the longitudinal axis and the tooling direction being essentially identical.

In an exemplary embodiment, the guide member comprises an end section having a guide bore for the tool, said guide bore being arranged laterally offset with respect to the guide member axis.

In another embodiment, the tool unit and the sleeve member comprise a locking mechanism locking the rotational position of the tool unit within the sleeve member in an axial end position of the tool unit, in particular to prevent the guide member from rotating when working a bone. In addition, this ensures a correctly positioned tool. In particular, the rotational locking is possible in at least two different rotational positions of the tool unit within the sleeve member with respect to the instrument axis, in particular said tool unit being rotated in one rotational position by 180° around the instrument axis with respect to another rotational position.

In another embodiment, the rotational position is adapted to be unlocked by an axial displacement of the tool unit towards the manipulation side of the tool unit thus enabling a rotational displacement of the tool unit within the sleeve member, whereby the tool unit remains at least partly received within the sleeve member.

In another embodiment, the locking mechanism comprises at least one locking slot arranged at one of the sleeve member and the tool unit, and at least one counterpart element adapted to fit into the locking slot free of clearance in the circumferential direction arranged on the other of the sleeve member and the tool unit, and wherein the locking slot is in particular arranged at and open towards one axial end of the sleeve member or the tool unit.

In another embodiment, the tool has a tool head adapted to work at least one workpiece, in particular a bone, down to a fixed worked depth, with the tool unit having a stop to fix the worked depth.

In another embodiment, the stop is made as a handle for the tool, said handle being fixedly connected to the tool.

In another embodiment, the sleeve member has a front part adapted to penetrate down to a fixed insertion depth into a cavity of a bone or a gap between two adjacent bones. In particular, the sleeve unit comprises a stop element which cooperates with the sleeve member, for example, to limit the insertion depth or worked depth of the front part of the sleeve member. Additionally, the sleeve unit may comprise adjustment means, which cooperate with the stop element and the sleeve member to adjust an axial position of the stop element relative to the sleeve member. Additionally, the adjustment means may comprise a thread provided at an outer surface of the sleeve member and an adjustment nut cooperating with said thread, said adjustment nut is suited for being displaced along the instrument axis via the thread.

In another embodiment, the stop element and the sleeve member are adapted to be rotationally and fixedly coupled to one another via a rotational lock to prevent rotation of the stop element about the central guide axis.

In another embodiment, the rotational lock comprises at least one guiding slot formed at one of the stop element and the sleeve member, or a front part thereof, and at least one counterpart element arranged at the other of the stop element and the sleeve member, or a front part thereof, the counterpart element being adapted to be received essentially free of clearance in circumferential direction within the at least one guiding slot.

In still another embodiment, the sleeve unit has at least one slot, at least one drill wire being suited to be introduced through the slot to fasten the sleeve unit to at least one bone.

In yet another embodiment, the instrument comprises a distractor unit to distract two bones, wherein the sleeve unit is suited for being guided on the distractor unit to position the sleeve unit between the two distracted bones.

The different aforesaid embodiments of an instrument in accordance with the independent instrument claim and the features realized there can naturally be combined with one another.

Further embodiments of the disclosure are also recited in the dependent claims, in the description and in the drawing.

Further areas of applicability of the present disclosure will become apparent from the detailed description provided hereinafter. It should be understood that the detailed description and specific examples are intended for purposes of illustration only and are not intended to limit the scope of the disclosure.

The present disclosure will become more fully understood from the detailed description and the accompanying drawings, wherein:
- Fig. 1: illustrates different views of a sleeve unit of the instrument.
- Fig. 2: illustrates different views of the sleeve unit of Fig. 1 with introduced distractor unit.
- Fig. 3: illustrates different views of a tool unit of the instrument.
- Fig. 4: illustrates different views of the sleeve unit of Fig. 1 with introduced tool unit of Fig. 3.
- Fig. 5: illustrates the arrangement of Fig. 4 in a cross-sectional side view.
- Fig. 6: is a side view of a distractor unit of the instrument.
- Fig. 7: is a perspective view of the sleeve unit of Fig. 1 in a disassembled state.

The following description of the embodiments is merely exemplary in nature and is in no way intended to limit the disclosure, its application, or uses.

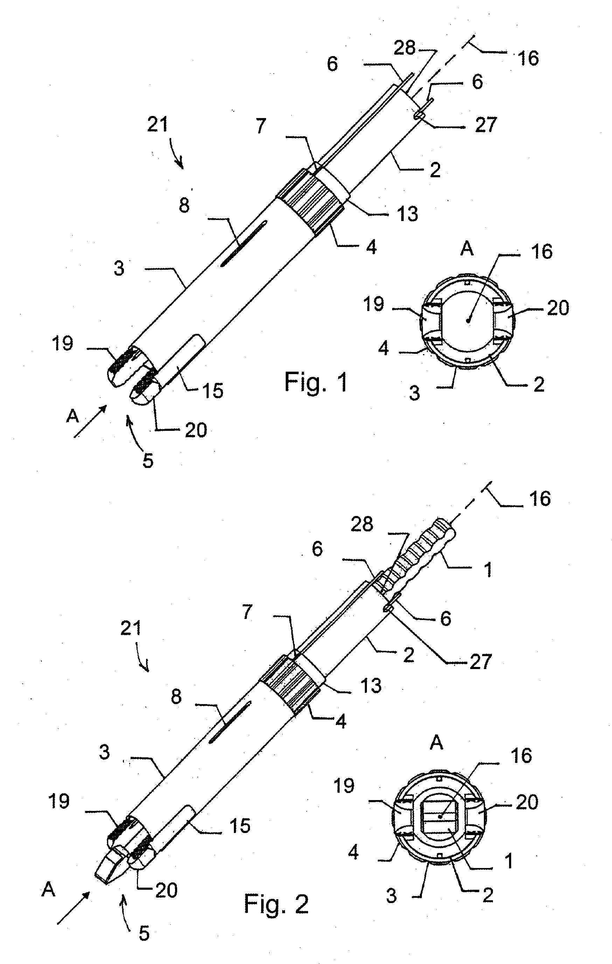

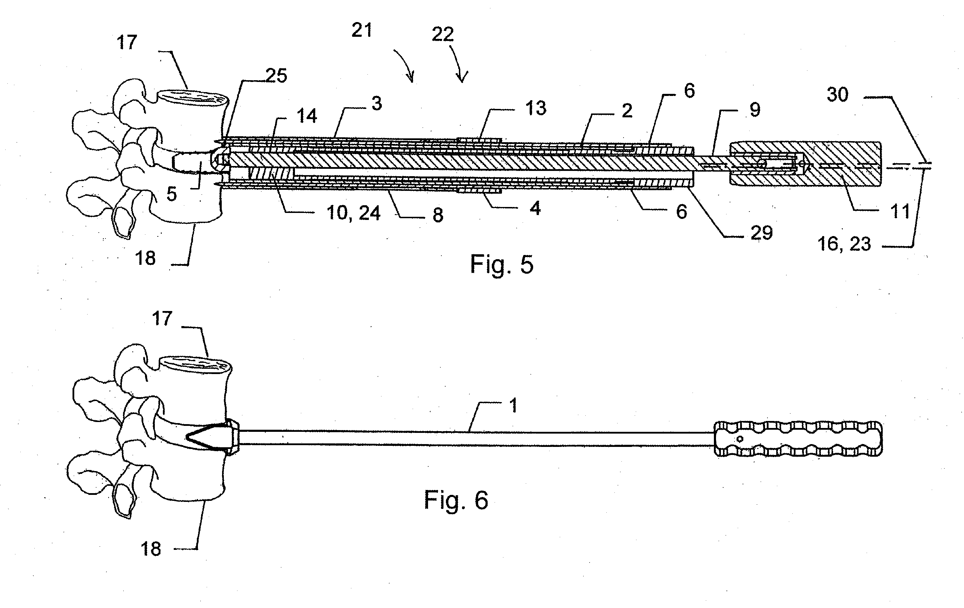

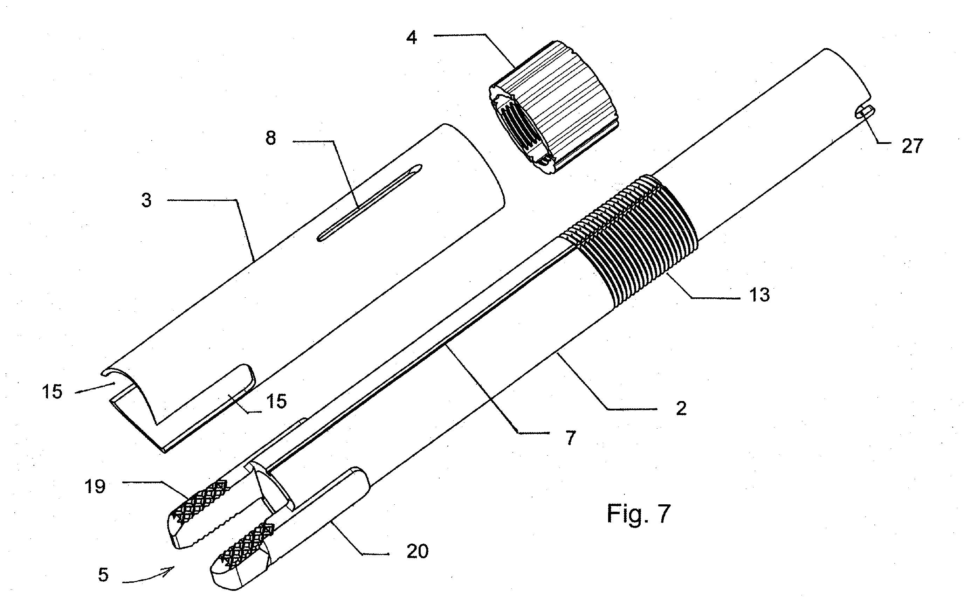

The instrument for the reaming of two adjacent vertebral bodies comprises a sleeve unit 21, as shown in Figs. 1, 2, 4 and 5. The sleeve unit 21 includes an essentially tubular sleeve member 2 having a passage, the passage defining an instrument axis 16, a tubular stop element 3 and an adjustment nut 4. The sleeve unit 21 is suited for being positioned between two vertebral bodies 17, 18, as shown in Fig. 5. In Fig. 7 the sleeve unit 21 is displayed in a disassembled state. In this embodiment, the instrument is a surgical instrument.

The sleeve member 2 terminates in a split front part 5 having two arms 19, 20 adapted to penetrate down to a fixed insertion depth between the two adjacent vertebral bodies 17, 18. The stop element 3 is arranged on the sleeve member 2 and adapted to restrict the insertion depth of the front part 5 of the sleeve member 2. To variably limit the insertion depth of the front part 5 of the sleeve member 2 the stop element 3 cooperates with the sleeve member 2 via the adjustment nut 4. The adjustment nut 4, which is adapted to abut the stop element 3, is suited for being displaced along the instrument axis 16 of the sleeve member 2 via a thread 13 provided at an outer surface of the sleeve member 2. Further, the two arms 19, 20 of the front part 5 of the sleeve member 2 are adapted to mesh with two guiding slots 15 formed at the stop element 3 and arranged on diametrically opposing sides with respect to the instrument axis 16 to prevent rotation of the stop element 3 about the instrument axis 16.

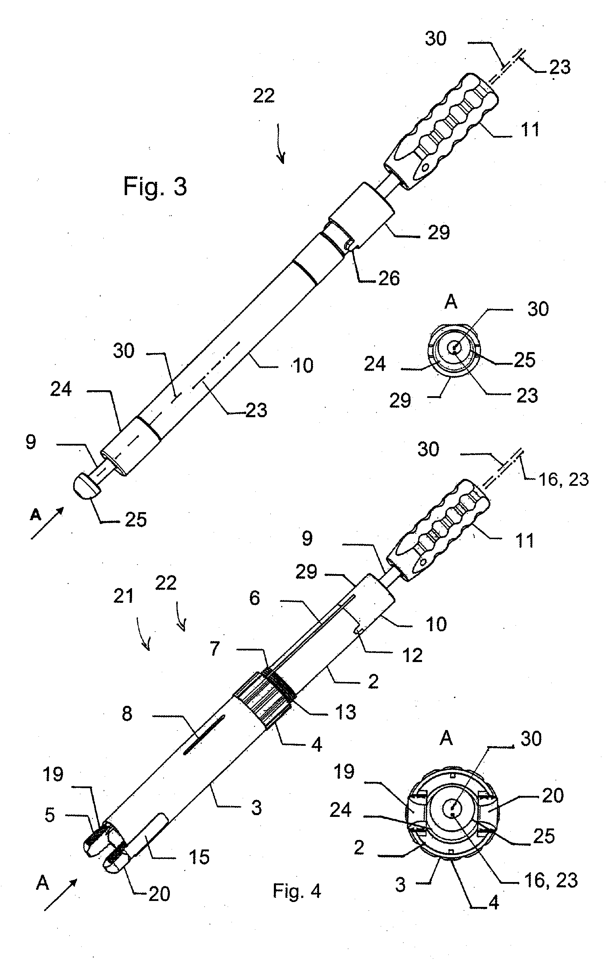

The instrument for the reaming of two adjacent vertebral bodies further comprises a tool unit 22 having a working side and a manipulation side, as shown in Figs. 3 to 5, which tool unit 22 can be introduced into and be slidably received within the passage of the sleeve member 2 as shown in Fig. 4 and 5. In particular, the working side is adapted to pass through the passage of the sleeve member 2. The tool unit 22 includes a tool 9, e.g., a reamer, which is rotatable about an axis of rotation 30 of the reamer 9, a tubular guide member 10 defining a guide member axis 23 and a handle 11 for the reamer 9, which handle 11 is fixedly connected to the reamer 9. The axis of rotation 30 of the reamer 9 and the guide member axis 23 run parallel and are offset to one another, as shown in Figs. 3 to 5. The tool unit 22 defines a tooling direction, in which tooling is effected. In particular, the tooling direction corresponds to the advance direction or feed direction of the tool 9 of the tool unit 22. In particular, the axis of rotation 30 points into a direction, which is identical to the tooling direction.

Thus, the tool unit 22 is suited for positioning the reamer 9 laterally offset, in particular eccentrically, with respect to the guide member axis 23. This is made possible by a guide bore 14 formed in an end section 24 of the guide member 10 and being arranged laterally offset, in particular eccentrically, with respect to the guide member axis 23, as shown in Fig. 5. The guide member 10 on the other hand is suited for being received within the passage of the sleeve member 2 concentrically with respect to the instrument axis 16, as shown in Figs. 4 and 5. Thus, the reamer 9 can be guided in the interior of the sleeve member 2 laterally offset, in particular eccentrically, with respect to the instrument axis 16. In an assembled state the guide member axis 23 coincides with the instrument axis 16.

Further, the reamer 9 has a reamer head 25 which is adapted to ream the vertebral bodies 17, 18 down to a fixed reamed depth, which reamed depth is reached when the handle 11 abuts a back part 29 of the guide member 10. The tool 9 further comprises a shaft or a tool axle, i.e. the tool 9 without the tool head 25. The tool axle is suited for being received within the guide member 10 laterally offset, in particular eccentrically, with respect to the guide member axis 23. In Figs. 3 to 5, the tool axle is rotatably and slidably received within the guide member 10. The tool axle comprises a longitudinal axis, which points into a direction, which is identical to the tooling direction.

The sleeve member 2 has two locking slots or female connectors 27 arranged on a back part 28 of the sleeve member 2 and on diametrically opposing sides with respect to the instrument axis 16, as shown in Figs. 1 and 2. The back part 29 of the guide member 10 is bulged and has two counterpart elements or male connectors 26 arranged on diametrically opposing sides with respect to the guide member axis 23 shown in Fig. 3. The sleeve member 2 and the guide member 10 can be rotationally and fixedly coupled to one another in an axial end position of the tool unit 22 within the sleeve member 2 via the male and female connectors 26, 27 which form a rotational lock 12, as shown in Fig. 4. The male connectors 26, 27 are adapted to fit into the locking slots 27 free of clearance in the circumferential direction arranged on the sleeve member 2. This makes it possible for the tool unit 22 to be positioned in two different relative positions to the sleeve member 2, wherein the tool unit 22 is rotated in the one relative position by 180° around the instrument axis 23 with respect to the other relative position.

The sleeve member 2 has two slots 7 arranged on diametrically opposing sides with respect to the instrument axis 16, as shown in Figs. 1, 2 and 4, and extending all the way from the thread 13 to the front part 5, as shown in Fig. 7. In addition, the stop element 5 has two slots 8, the slots 8 being essentially in alignment with the slots 7. The slots 7 and 8 are provided to make it possible for two drill wires 6 shown in Figs. 1, 2, 4 and 5 to be introduced to fasten the sleeve unit 21 to the vertebral bodies 17, 18, as shown in Fig. 5.

The instrument for the reaming of two adjacent vertebral bodies further comprises a distractor unit 1 shown in Figs. 2 and 6. The distractor unit 1 is provided to distract the two vertebral bodies 17, 18 before positioning the sleeve unit 21 between the two vertebral bodies 17, 18, as shown in Fig. 6, the sleeve unit 21 being able to be guided on the distractor unit 1.

In an exemplary method for the reaming of two adjacent vertebral bodies the two vertebral bodies 17, 18 are distracted by means of the distractor unit 1 inserted from an anterior direction, as shown in Fig. 6, after the intervertebral disc between the two vertebral bodies 17, 18 has been removed. Subsequently, the sleeve unit 21 is guided on the distractor unit 1, as shown in Fig. 2, to position the sleeve unit 21 between the two vertebral bodies 17, 18, as shown in Fig. 5. The front part 5 of the sleeve member 2 penetrates down to a limited insertion depth. The front part 5 acts as a spacer for the two vertebral bodies 17, 18. The positioning nut 4 and the stop element 3 ensure the correct insertion depth of the front part 5 of the sleeve member 2 between the vertebral bodies 17, 18. The insertion depth is adjusted by displacing the positioning nut 4 along the instrument axis 16 via the thread 13 provided at the sleeve member 2. The stop element 3 is secured against rotation by means of the two guiding slots 15 formed at the stop element 3, which guiding slots 15 mesh with the two arms 19, 20 of the front part 5 of the sleeve member 2. After positioning the sleeve member 2, the distractor unit 1 is removed. Then the drill wires 6 are introduced through the slots 7 formed at the thread 13 until they enter the vertebral bodies 17, 18 to fasten the sleeve unit 21 to the vertebral bodies 17, 18, as shown in Fig. 5, to avoid lateral displacement of the sleeve unit 21. Further, the drill wires 6 are provided to avoid lifting of the vertebral bodies 17, 18 from the front part 5 of the sleeve member 2 when reaming. Alternatively, the drill wires 6 can be introduced through the slots 8 formed at the stop element 3 and be received by the slots 7 formed at the sleeve member 2.

Subsequently, the reamer 9 is introduced into the guide member 10. Afterwards, the handle 11 is put onto the reamer 9. Alternatively, the tool unit 22, including the reamer 9, the guide member 10 and the handle 11 as shown in Fig. 3, can be assembled before positioning the sleeve unit 21 between the vertebral bodies 17, 18 or even before distracting the vertebral bodies 17, 18. The assembled tool unit 22 is then introduced into the sleeve unit 21 in a first relative position, as shown in Fig. 5, until the bulged back part 29 of the guide member 10 abuts the back part 28 of the sleeve member 2, as shown in Fig. 4. At the same time the male connectors 26 of the guide member 10 catch the female connectors 27 of the sleeve member 2 to form the rotational lock 12 to prevent the guide member 10 from rotating with respect to the sleeve member 2.

The cranial vertebral body 17 is then reamed by means of the reamer 9 by, for example, manual clockwise rotation of the handle 11 of the tool unit 22, while slightly forcing the reamer 9 in a posterior direction. The fixed reamed depth is reached when the handle 11 abuts the bulged back part 29 of the guide member 10. To allow rotation of the tool unit 22 about the guide member axis 23 of the guide member 10, the reamer 9 is retracted from the cranial vertebral body 17 and the guide member 10 is retracted at least partly from the sleeve member 2 such that the rotational lock 12 unlocks. The tool unit 22 is then rotated by 180° about the guide member axis 23. Subsequently, the tool unit 22 is again fully introduced into the sleeve unit 21 in a second relative position (not shown) and the caudal vertebral body 18 is reamed by means of the reamer 9.

In an exemplary method for the implantation of an implant between two adjacent vertebral bodies, an implant is inserted between the two reamed vertebral bodies 17, 18 after reaming the two adjacent vertebral bodies 17, 18. The implant may be inserted between the two vertebral bodies 17, 18 through the sleeve unit 21 or without the aid of the sleeve unit 21 after the sleeve unit 21 is removed.

The description of the disclosure is merely exemplary in nature and, thus, variations that do not depart from the gist of the disclosure are intended to be within the scope of the disclosure. Such variations are not to be regarded as a departure from the spirit and scope of the disclosure.

An exemplary method for the working of at least one bone comprises choosing an instrument in accordance with an instrument as mentioned above; positioning the sleeve unit at the at least one bone; introducing the tool unit into the sleeve unit in a first relative position; working the at least one bone by means of the tool; readjusting the position of the tool unit within the sleeve unit to a second relative position different than the first relative position; and working the at least one bone by means of the tool.

In one embodiment, the method further comprises introducing at least one drill wire through at least one slot formed at the sleeve unit before the introduction of the tool unit into the sleeve unit; and fastening the sleeve unit to the at least one bone by means of the at least one drill wire.

In a further embodiment, the method further comprises distracting two bones by means of a distractor unit of the instrument before the positioning of the sleeve unit; guiding the sleeve unit on the distractor unit to position the sleeve unit between the two bones; and removing the distractor unit after the positioning of the sleeve unit.

In another embodiment, the method further comprises fixing the insertion depth of a front part of the sleeve member into the at least one bone.

In still another embodiment, the method further comprises rotating the tool unit around an axis of the tool unit by 180° with respect to the first relative position before readjusting to the second relative position.

In yet another embodiment, the method further comprises leaving the sleeve unit in position throughout the execution of the method steps following the step of positioning the sleeve unit.

A method for the implantation of an implant between two bones comprises working the two bones in accordance with a method as mentioned above; and inserting an implant between the two worked bones.

A method for the reaming of two adjacent vertebral bodies comprises choosing an instrument in accordance with an instrument as mentioned above; positioning the sleeve unit between the two vertebral bodies; introducing the tool unit into the sleeve unit in a first relative position; reaming the one of the two vertebral bodies by means of the reamer; readjusting the position of the tool unit within the sleeve unit to a second relative position different than the first relative position; and
reaming the other of the two vertebral bodies by means of the reamer.

In one embodiment, the method further comprises introducing at least one drill wire through at least one slot formed at the sleeve unit before the introduction of the tool unit into the sleeve unit; and fastening the sleeve unit to at least one vertebral body by means of the at least one drill wire.

In a further embodiment, the method further comprises distracting the two vertebral bodies by means of a distractor unit of the reaming instrument before the positioning of the sleeve unit; guiding the sleeve unit on the distractor unit to position the sleeve unit between the two vertebral bodies; and removing the distractor unit after the positioning of the sleeve unit.

In another embodiment, the method further comprises fixing the insertion depth of a front part of the sleeve member between the two vertebral bodies.

In still another embodiment, the method further comprises rotating the tool unit around an axis of the tool unit by 180° with respect to the first relative position before readjusting to the second relative position.

In yet another embodiment, the method further comprises leaving the sleeve unit in position throughout the execution of the method steps following the step of positioning the sleeve unit.

A method for the implantation of an implant between two adjacent vertebral bodies comprises reaming the two adjacent vertebral bodies in accordance with a method as mentioned above; and inserting an implant between the two reamed vertebral bodies.

### Reference Numeral List

- 1: distractor unit
- 2: sleeve member
- 3: stop element
- 4: positioning nut
- 5: front part of the sleeve member
- 6: drill wire
- 7: slot formed at the sleeve member
- 8: slot formed at the stop element
- 9: reamer
- 10: guide member
- 11: handle
- 12: rotational lock
- 13: thread
- 14: guide bore
- 15: guiding slot formed at the stop element
- 16: instrument axis
- 17: cranial vertebral body
- 18: caudal vertebral body
- 19: arm of the front part
- 20: arm of the front part
- 21: sleeve unit
- 22: tool unit
- 23: guide member axis
- 24: end section
- 25: reamer head
- 26: male connector
- 27: female connector
- 28: back part of the sleeve member
- 29: bulged back part of the guide member
- 30: axis of rotation of the reamer

## Claims

1. An instrument, comprising:
a tool unit (22) including a tool (9), and
a sleeve unit (21) including a sleeve member (2) having a passage, said passage defining an instrument axis (16),
wherein the tool unit (22) is suited for being movably and/or slidably received within the passage of the sleeve member (2) and for positioning the tool (9) laterally offset with respect to the instrument axis (16),
wherein the tool unit (22) has a working side and a manipulation side, the working side being adapted to pass at least partially through the passage of the sleeve member (2), and
wherein the tool unit (22) is suited for arranging the tool (9) within the passage of the sleeve member (2) in at least two different rotational positions with respect to the instrument axis (16).

2. An instrument in accordance with claim 1, **characterized in that** the tool unit (22) has a tooling direction in which tooling is effected, which tooling direction is inclined with respect to the instrument axis (16) by not more than 15°, in particular not more than 10°, and in particular is essentially parallel to the instrument axis.

3. An instrument in accordance with claim 1 or 2, **characterized in that** the tool unit (22) comprises a guide member (10), said guide member (10) having a guide member axis (23), wherein the guide member (10) is suited for being slidably received within the passage of the sleeve member (2) and for positioning the tool (9) laterally offset with respect to the guide member axis (23).

4. An instrument in accordance with claim 3, **characterized in that** the tool (9) comprises a tool axle, which is suited for being received within the guide member (10) laterally offset with respect to the guide member axis (23).

5. An instrument in accordance with claim 4, **characterized in that** the tool axle is rotatably and/or movably and/or slidably received within the guide member (10).

6. An instrument in accordance with claim 4 or claim 5, wherein the tool unit (22) has a tooling direction in which tooling is effected **characterized in that** the tool axle comprises a longitudinal axis, the longitudinal axis and the tooling direction being essentially identical.

7. An instrument in accordance with at least one of the claims 3 to 6, **characterized in that** the guide member (10) comprises a guide bore (14) for the tool (9), said guide bore (14) being arranged laterally offset with respect to the guide member axis (23).

8. An instrument in accordance with at least one of the preceding claims, **characterized in that** the tool unit (22) and the sleeve member (2) comprise a locking mechanism (12) locking the rotational position of the tool unit (22) within the sleeve member (2) in an axial end position of the tool unit (22).

9. An instrument in accordance with claim 8, **characterized in that** the rotational locking is possible in at least two different rotational positions of the tool unit (22) within the sleeve member (2) with respect to the instrument axis (16), in particular said tool unit (22) being rotated in one rotational position by 180° around the instrument axis (16) with respect to another rotational position.

10. An instrument in accordance with claim 8 or claim 9, **characterized in that** the rotational position is adapted to be unlocked by an axial displacement of the tool unit (22) towards the manipulation side of the tool unit (22) thus enabling a rotational displacement of the tool unit (22) within the sleeve member (2), whereby the tool unit (22) remains at least partly received within the sleeve member (2).

11. An instrument in accordance with any of claims 8 to 10, wherein the locking mechanism comprises at least one locking slot (27) arranged at one of the sleeve member (2) and the tool unit (22), and at least one counterpart element (12) adapted to fit into the locking slot (27) free of clearance in the circumferential direction arranged on the other of the sleeve member (2) and the tool unit (22), and wherein the locking slot (27) is in particular arranged at and open towards one axial end of the sleeve member (2) or the tool unit (22).

12. An instrument in accordance with at least one of the preceding claims, **characterized in that** the tool (9) has a tool head (25) adapted to work at least one workpiece, in particular a bone, (17, 18) down to a fixed worked depth, with the tool unit (22) having a stop (11) to fix the worked depth.

13. An instrument in accordance with claim 12, **characterized in that** the stop (11) is made as a handle for the tool (9), said handle (11) being fixedly connected to the tool (9).

14. An instrument in accordance with at least one of the preceding claims, **characterized in that** the sleeve member (2) has a front part (5) adapted to penetrate down to a fixed insertion depth into a cavity of a bone or a gap between two adjacent bones (17, 18).

15. An instrument in accordance with at least one of the preceding claims, **characterized in that** the sleeve unit (21) comprises a stop element (3), which cooperates with the sleeve member (2).

16. An instrument in accordance with claim 15, **characterized in that** the sleeve unit (21) comprises adjustment means (4, 13), which cooperate with the stop element (3) and the sleeve member (2) to adjust an axial position of the stop element (3) relative to the sleeve member (2).

17. An instrument in accordance with claim 16, **characterized in that** the adjustment means (4, 13) comprise a thread (13) provided at an outer surface of the sleeve member (2) and an adjustment nut (4) cooperating with said thread (13), said adjustment nut (4) is suited for being displaced along the instrument axis (16) via the thread (13).

18. An instrument in accordance with at least one of the claims 15 to 17, **characterized in that** the stop element (3) and the sleeve member (2) are adapted to be rotationally and fixedly coupled to one another via a rotational lock (15).

19. An instrument in accordance with claim 18, **characterized in that** the rotational lock (15) comprises at least one guiding slot formed at one of the stop element (3) and the sleeve member (10), or a front part (5) thereof, and at least one counterpart element (19, 20) arranged at the other of the stop element (3) and the sleeve member (10), or a front part (5) thereof, the counterpart element (19, 20) being adapted to be received essentially free of clearance in circumferential direction within the at least one guiding slot (15).

20. An instrument in accordance with at least one of the preceding claims, **characterized in that** the sleeve unit (21) has at least one slot (7, 8), at least one drill wire (6) being suited to be introduced through the slot (7, 8) to fasten the sleeve unit (21) to at least one bone (17, 18).

21. An instrument in accordance with at least one of the preceding claims, **characterized in that** the instrument comprises a distractor unit (1) to distract two bones (17, 18), wherein the sleeve unit (21) is suited for being guided on the distractor unit (1) to position the sleeve unit (21) between the two distracted bones (17, 18).
